# EUROPEAN PATENT SPECIFICATION

(11) **EP 3 927 801 B1**
(45) Date of publication and mention of the grant of the patent: **04.11.2026**
(21) Application number: 20711735.9
(22) Date of filing: 20.02.2020
(51) Int. Cl.: C11D 3/00, C11D 3/10, C11D 3/20, C11D 11/00, C11D 17/00, C11D 17/06

(54) **STABLE ANHYDROUS FOAMING AND GELLING HAND SOAP CONCENTRATE AND METHOD OF MAKING SAME**
STABILES WASSERFREIES SCHÄUMENDES UND GELIERENDES HANDSEIFENKONZENTRAT UND VERFAHREN ZUR HERSTELLUNG DAVON
CONCENTRÉ DE SAVON POUR LES MAINS À FORMATION DE GEL ET DE MOUSSE, STABLE, ANHYDRE ET SON PROCÉDÉ DE PRODUCTION

(30) Priority: 20.02.2019 US 201962808021 P; 20.08.2019 US 201962889480 P
(43) Date of publication of application: 29.12.2021
(73) Proprietor: One Home Brands, Inc., New York, NY 10024-4926 (US)
(72) Inventor: NAQVI, Syed Humza, Walnut Creek, California 94597 (US)
(74) Representative: Müller-Boré & Partner Patentanwälte PartG mbB
(86) International application number: PCT/US2020/019061
(87) International publication number: WO 2020/172423

(56) References cited:
- EP-A2- 0 370 969
- EP-A2- 1 577 375
- WO-A1-2018/141074
- CN-A- 106 038 352

## Description

### CROSS REFERENCE TO RELATED APPLICATIONS

This application claims priority to U.S. Provisional Application Nos. 62/808,021, filed February 20, 2019, and 62/889,480, filed August 20, 2019.

### BACKGROUND

The majority of Hand Soap products come in as vessel with a dispenser. They also can come in as bulk as refill. These vessels are mostly plastic, glass, carboard lined with plastic as water barrier. Majority of time these soap in the market are in thin liquid or gel forms. One of the problem is the packaging. Single use plastic is everywhere and it is wreaking havoc on the environment. Only 9% of all plastic is actually recycled, and packaging generates the largest portion of municipal waste (~30%). Packaged products are inefficient for businesses and the people who buy them. WO 2018/141074 A1 describes a method for removing milk residues, and composition of a cleaning tablet. EP 1 577 375 A2 describes a hand soap product and hand soap dispenser.

Removing the water from Hand Soap formulations removes the need for single use plastic packaging and the waste that comes with it, such as packaging waste, product waste, and the waste of resources used to ship water.

Thus, a need exists for new stable formulations of hand soap concentrate that meet the needs of consumers, while also reducing the amount of waste generated in their production and shipping.

### SUMMARY OF THE INVENTION

The invention is defined in the appended claims.

### DETAILED DESCRIPTION OF THE INVENTION

This disclosure relates to a stable, anhydrous hand soap concentrate formulation in a solid form. The inventors have discovered a solid formulation that is both good for the environment and effective for cleaning. The advantages of this anhydrous solid form over the traditional liquid cleansers include chemical stability, reduced packaging, and convenience for the consumer. The stable, anhydrous hand soap concentrate formulation can be in a tablet, granulars, powder, sachet, or polymer membrane (PVA, PVP, HPMC, etc) form.

As used in this specification, the singular forms "a," "an," and "the" include plural referents unless the context clearly dictates otherwise. Thus, for example, reference to a "preservative booster" includes a single kind of preservative booster or two or more different kinds of preservative booster.

"About" as used herein means within an acceptable error range for the particular value as determined by one of ordinary skill in the art, which will depend in part on how the value is measured or determined, (*i.e*., the limitations of the measurement system). For example, "about" can mean within 1 or more than 1 standard deviations, per practice in the art. Where particular values are described in the application and claims, unless otherwise stated, the term "about" means within an acceptable error range for the particular value. The term "about" when qualifying a value of a stated item, number, percentage, or term refers to a range of plus or minus ten percent of the value of the stated item, percentage, parameter, or term.

The term "anhydrous" as used herein refers to a stable, anhydrous hand soap concentrate formulation comprising less than about 5%, 4%, 3%, 2% or 1% by weight of water based on the weight of the concentrate formulation.

The term "substantially fatty acid-free" as used herein refers to a stable, anhydrous hand soap concentrate formulation comprising less than 10%, 9%, 8%, 7%, 6%, 5%, 4%, 3%, 2%, or 1% by weight of a fatty acid (or salt thereof) based on the weight of the concentrate formulation, or comprising a fatty acid (or salt theref) in an amount less than the amount used in a hand soap bar.

The term "substantially animal fat free" as used herein refers to a stable, anhydrous hand soap concentrate formulation comprising less than 10%, 9%, 8%, 7%, 6%, 5%, 4%, 3%, 2%, or 1% by weight of an animal fat (such as tallow) (or salt thereof) based on the weight of the concentrate formulation, or comprising or comprising a an animal fat (or salt theref) in an amount less than the amount used in a hand soap bar.

The term "water softening agent" as used herein may be used interchangeably with chelating agent. For example citric acid and ethylenediaminetetraacetic acid (EDTA) are examples of water softening agent.

Some of the ingredients may have multiple functions. However, when two or more ingredients defined based on their functions are included in a formulation disclosded herein, the ingredients differ from each other in terms of their chemical structure. For example citric acid can be a water softening agent and a acidic cleaner as well, but when both water softening agent and acidic cleaner are used in the description of the formulation, they intend to refer to different ingredients in terms of the chemical structure.

The term "comprising" includes the embodiments of "consisting of" or "consisting essentially of."

The stable anhydrous hand soap concentrate formulation in a solid form according to the claimed invention comprises a surfactant and a pH control agent. The pH control agent may be present in an amount greater than the amount of the surfactant. The stable anhydrous hand soap concentrate formulation in a solid form further comprises a preservative and a fragrance and may comprise an ingredient selected from a preservative booster, a water softening agent, an emolient, a viscosity adjuster, an acidic cleaner, a basic cleaner, a thickening agent, and a binding agent. The stable anhydrous hand soap concentrate formulation in a solid form is substantially fatty acid free and/or substantially animal fat free. The preservative is present in an amount ranging from about 10% to about 30%, from about 10% to about 25%, or from about 10% to about 20%, by weight, based on the weight of the formulation.

In one aspect described herein in general, a stable anhydrous foaming and gelling hand soap concentrate tablet (also referred to foaming hand soap concentrate tablet or stable anhydrous hand soap concentrate tablet) is provided. The foaming hand soap concentrate tablet of this disclosure may include an acidic cleaner, a binding agent, a surfactant, and optionally a pH control agent. The binding agent and the surfactant function as a solvent and oily soil remover, respectively, when the tablet is dissolved in water before use. The foaming hand soap concentrate tablet is substantillay fatty acid free or substantially animal fat free. The effervescent ingredients (e.g, the acidic and basic cleaner) can facilitate homogeneous distribution and dissolution of the surfactant into water before use.

The embodiments described herein for foaming hand soap concentrate tablet can also apply to the stable anhydrous hand soap concentrate formulation in a solid form.

In some embodiments, the foaming hand soap concentrate tablet may further comprise a pH control agent.

In some embodiments, the foaming hand soap concentrate tablet may further comprise a chelating agent.

In some embodiments, the foaming hand soap concentrate tablet may further comprise a lubricating agent.

In some embodiments, the foaming hand soap concentrate tablet may further comprise a pH control agent, a chelating agent, and a lubricating agent.

In some embodiments, the foaming hand soap concentrate tablet may further comprise a carrier.

In some embodiments, the foaming hand soap concentrate tablet may further comprise a pH control agent, a chelating agent, a lubricating agent, and a carrier.

In some emboidments, the foaming hand soap concentrate tablet may further comprise a process aid/emollient.

In each of the preceeding embodiments, the foaming hand soap concentrate tablet may have a pH of about 4.0 to about 6.0 when dissolved in appropriate amount of water.

In each of the preceeding aspect or embodiments, the foaming hand soap concentrate tablet may further comprise a thickening agent. The thickening agent provides at least one of the desired lathering properties, viscocity properties, emollient properites, increased volume of foam, and retained foam formation.

In each of the preceeding aspect or emboidments, the foaming hand soap tablet may further comprise a basic cleaner.

In each of the preceeding aspect or emboidments, the foaming hand soap concentrate tablet may further comprise a preservative and optionally a preservative booster.

In each of the preceeding aspect or emboidments, the foaming hand soap concentrate tablet may further comprise a dye/colorant and optionally a fragrance.

In each of the preceeding aspect or emboidments, the foaming hand soap concentrate tablet may further comprise a water softening agent.

In each of the preceeding aspect or embodiments, the surfactant can be selected from non-ionic and anionic surfactants.

In some embodiments, the pH control agent is not the acidic or basic cleaner. When both an acidic or basic cleaner and a pH control agent are contained in a tablet, the acidic or basic cleaner differs from the pH control agent.

In some embodiments, the foaming hand soap concentrate tablet comprises a surfactant, a binding agent, a thickening agent, a preservative, a preservative booster, a cleaner, a process aid/emollient, and optionally a fragrance.

In some embodiments, the foaming hand soap concentrate tablet comprises citric acid, sodium coco sulfate, sodium carbonate, sodium benzoate, sodium alginate, PEG (polyethylene glycol such as PEG 8000), sorbitol crystalline p20, potassium sorbate, medium-chain triglycerides oil, and optionally a fragrance.

In some embodiments, the foaming hand soap concentrate tablet comprises a surfactant that is a combination of a nonionic surfactant and a nonionic surfactant, a binding agent, a cleaner, and optionally a colorant.

In some emboidments, the the foaming hand soap concentrate tablet comprises citric acid, sodium carbonate, sodium lauryl sulfate, ethoxylated alcohol, PEG (such as PEG 8000), and optinally a colorant.

In some emboidments, the foaming hand soap tablet may include a pH control agent, an anionic surfactant, a binding agent, a preservative, and a lubricating agent. In some emboidments, the foaming hand soap tablet may include a pH control agent, non-ionic surfactant, a binding agent, a preservative, and a lubricating agent. The foaming hand soap tablet may have a pH of about 4.0 to about 6.0 when dissolved in appropriate amount of water. In one embodiment, the foaming hand soap tablet includes citric acid, malic acid, sodium bicarbonate, sodium coco sulfate, dextrose, polyethylene glycol, a preservative, and medium chain triglyceride oil powder. The foaming hand soap tablet may also include a fragrance.

In each of the preceeding embodiments or aspects, the tablet may further contain biologic cleaners, such as enzymes (e.g., protease, amylase, lipase, cellulose, pectinase, mannanase, and the like) and probiotics (e.g., lactobacillus, bifidobacterial, and the like). Biological cleaners may be present in an amount of about 0.01% to about 50% by weight, based on the weight of the tablet.

The amount of acidic cleaner in the tablet may range from about 1% to about 85%, from about 15% to about 20%, from about 20% to about 60%, from about 20% to about 45%, or from about 25% to about 35%, by weight, based on the weight of the tablet. The amount of acidic cleaner per tablet may be about 1%, about 5%, about 10%, about 15%, about 20%, about 25%, about 30%,about 35%, about 40%, about 50%, about 60%, about 70%, about 75%, about 80%, about 85%, about 5% to about 85%, about 10% to about 75%, about 15% to about 70%, about 20% to about 65%, about 25% to about 60%, about 30% to about 55%, about 35% to about 50%, or about 40% to about 45%. The acidic cleaner may be citric acid and/or malic acid.

The amount of the basic cleaner in the tablet may range from about 5 % to about 40%, from about 5 % to about 30 %, from about 10% to about 30%, from about 10% to about 25%, from about 5% to about 10%, from about 40% to about 60%, or from about 35% to about 45%, by weight, based on the weight of the tablet. The amount of acidic cleaner per tablet may be about about 5%, about 7%, about 9%, about 11%, about 13%, about 15%,about 17%, about 19%, about 21%, about 23%, about 25%, about 27%, about 29%, about 31%, about 33%, about 35%, about 37%, about 39%, or about 40%. The basic cleaner may be sodium carbonate, sodium bicarbonate and/or any other alkali carbonates.

The amount of the preservative in the tablet may range from about 5 % to about 40%, from 5% to about 30 %, from about 10% to about 30%, from about 10% to about 25%, or from about 10% to about 20%, by weight, based on the weight of the tablet. The amount of the preservative per tablet may be about 5%, about 7%, about 9%, about 11%, about 13%, about 15%,about 17%, about 19%, about 21%, about 23%, about 25%, about 27%, about 29%, about 31%, about 33%, about 35%, about 37%, about 39%, or about 40%. The preservative may be sodium benzoate, potassium sorbate, gluconolactone, and/or biocideal preservatives.

The amount of the preservative booster in the tablet may range from about 0.1 % to about 10%, from about 0.5 % to about 10 %, from about 1 % to about 10 %, or from about 1% to about 5%, by weight, based on the weight of the tablet. The amount of the preservative booster per tablet may be about 0.1%, about 0.2 %, about 0.3%, about 0.4%, about 0.5%, about 1%,about 2%, about 3%, about 4%, about 5%, about 6%, about 7%, about 8%, about 9%, or about 10%. The preservative booster may be a sorbate such as potassium sorbate.

The amount of the thickening agent in the tablet may range from about 1% to about 15%, from about 1% to about 10%, or from 5% to about 10 %, by weight, based on the weight of the tablet. The amount of the thickening agent per tablet may be about 1%, about 2%, about 3%, about 4%, about 5%, about 6%,about 7%, about 8%, about 9%, about 10%, about 11%, about 12%, about 13%, about 14%, or about 15%, by weight, based on the weight of the tablet. The thickening agent may be xanthum gum (such as sodium alginate, alginate SS207), NaCl, KCl , potassium alginate, gura gum, and/or HPMC. Thickening agent can bring emollient properties and add body to the foam. Without thickening agent, the foam would go flat as the hands are rubbed for cleaning. Thickening agent can also provide the foam with paste like structure and when combined with surfactant it actually provides lathering properties and allows the foam to retain itself.

The pH of the tablet dissolved in water may range from about 4.5 to about 5.5. The pH of the tablet dissolved in appropriate amount of water may be about 4.5, about 5.0, or about 5.5. The pH may be adjusted by acidic or basic agent used in the tablet, such as the acidic cleaner and/or basic cleaner such as sodium carbonate or citric acid. The pH can also be adjusted by a pH control agent that differs from the acidic cleaner or basic cleaner contained in the tablet.

The pH control agent is in an amount sufficient to adjust the pH when dissolved in water from about 4.5 to about 5.5. When the pH control agent differs from the acidic or basic cleaner, its amount in the tablet ranges from about 5% to about 15%, such as from about 8% to about 12% by weight, based on the weight of the tablet. The pH control agent may be sodium metasilicate.

The amount of chelating agent in the tablet may range from about 0.01% to about 95% by weight, based on the weight of the tablet. The amount of chelating agent may be about 1%, about 1.5%, about 2.0%, about 2.5%, about 3.0%, about 3.5%, about 4.0%, about 4.5%, about 5.0%, about 5.5%, about 6.0%, about 6.5%, about 7.0%, about 7.5%, about 8.0%, about 8.5%, about 9.0%, about 9.5%, about 10.0%, about 2% to about 10.0%, about 3% to about 7%, or about 1% to about 10% by weight. The cleaning tablet may contain one or more chelating agents, such as MGDA (methylglycineadiacetic acid and salts), tri-sodium citrate, GLDA (L-glutamic acid, N, N-diacetic acid sodium salts), EDDS (ethylenediaminedisuccinic acid and salts), and IDS (iminodisuccinic acid and salts).

The amount of the binding agent in the tablet may range from about 0% to about 30% such as less than 5%, from about 0% to about 10%, from about 0% to about 20%, from about 3% to about 8%, from about 5% to about 15% by weight, based on the weight of the tablet. The amount of binding agent is suitable to form a tablet and may be about 1%, about 2%, about 3%, about 4%, about 5%, about 6%, about 7%, about 8%, about 9%, about 10%, about 12%, about 15%, about 18%, about 20%, about 3% to about 7%, about 4% to about 8%, about 5% to about 10%, or about 10% to about 20%. The binding agent may be selected from polyethylene glycol (e.g., polyethylene glycol 8000 such as T-Det^{®} PEG 8000P) and sorbitol (such as sorbitol crystalline P20) and dextrose.

The amount of surfactant (i.e., oily soil remover) in the tablet may range from about 0.01% to about 40%, from about 1% to about 20%, from about 10% to about 20%, from about 10% to about 25%, from about 10% to about 40%, or from about 15% to about 25%, by weight, based on the weight of the tablet. The amount of oily soil remover may be about 1%, about 2%, about 3%, about 4%, about 5%, about 6%, about 7%, about 8%, about 9%, about 10%, about 12%, about 15%, about 18%, about 20%, about 16% to about 20%, about 3% to about 7%, about 4% to about 8%, about 8% to about 12%, about 5% to about 10%, about 10% to about 15%, or about 10% to about 20%. The surfactant can be natural or synthetic surfactants, such an anionic, nonionic, amphoteric, zwitterioinic, or cationic surfactants, such as anionic and non-ionic surfactants, further such as a surfactant selected from sodium coco sulfate, ethoxylated alcohols (such as ethoxylated alcohol C(10-12)-C(14-16) with 4-8 moles ethoxylation, for example Clariant Genapol LA 060 (ethoxylated alcohol C12-C16) w/ 6 moles ethoxylation, ethoxylated alcohols C8-C10 6-8 moles of EO, etc.), sodium lauryl sulfate, and alkyl polyglucosides (such as lauryl glucoside, caprylyl / myristyl glucoside, caprylyl / decyl Glucoside).

The amount of the process aid/emollient in the tablet may range from about 0.1% to about 1%, such as from about 0.1% to about 0.7%, further such as from about 0.2% to about 0.6% by weight, based on the weight of the tablet. The process aid/emollient can be selected from medium chain triglycerides with two or three fatty acids having an aliphatic tail of 6-12 carbon atoms, coconut fatty acid, coconut oil, glycerin, esters, and plant base oils.

The amount of the lubricating agent in the tablet may range from about 0.1 to about 2.0% by weight based on the weight of the tablet. Exemplary lubricating agent can be selected from magnesium stearate, leucine, sodium lauryl sulfate, sodium benzoate etc.

The amount of the carrier in the tablet may range from about 1% to about 10% by weight based on the weight of the tablet. Exemplary carrier can be selected from silicon dioxide.

The fragrance in the tablet may be natural fragrances (e.g., essential oils) and/or synthetic fragrances and perfumes in the form of oils, crystals, powders, granules, and encapsulations. The fragrance can be selected from fragrance clean basil.

The dye or coloring agent can be any of the Food, Drug and Cosmetic (FD&C) approved dyes and colorants.

The foaming hand soap concentrate tablet may also be formulated in the form of a sheet.

The foaming hand soap concentrate tablet can be in any size. For example, the tablet can be in any size that when dissolved into 355 mL (12 oz) or less amount of water, a fully functional foaming hand soap for multiple use is formed. The tablet may weigh from about 5.0 to about grams 15grams, from about 8.2 to about 8.8 grams, such as about 8.5 grams and dissolved into about 207 to 296 mL (7 to 10 oz), such as about 266 mL (9 oz), of water before use.

To avoid effervescence from happening during storage when the tablets contain both an acidic and basic cleaner, the tablet can have a sufficient hardness and/or the tablet contains a desiccating agent such as hydrated silica or any other agent known to absorb moisture.

### Methods for Preparing Stable Anhydrous Hand Soap Concentrate Tablets

The stable anhydrous hand soap tablets can be prepared using any suitable method. Stable anhydrous Hand Soap cleanser tablet can be prepared using direct compression or wet granulation process. For this application direct compression is most preferred. The term direct compression (or direct compaction) is used to define the process by which tablets are compressed directly from powdered mixture of ingredients into a firm compact without employing the process of granulation. Powder is blended homogeneously by using a blender (Ribbon Blender, V-blender, paddle blender, drum mixing). The powder blender is then charged into the hopper of tablet press. Desire weight, compression ton, & hardness of tablet are set as the tablets get compressed and come out of the tablet press.

### Formats

Although described throughout the application as tablets, the stable anhydrous hand soap concentrate formulations may be formatted as tablets, powders, granules, sachets, packs, polymer membrane (natural or syntactic) and sheets. The stable anhydrous hand soap concentrate formulations may also be formatted in single-use sheets.

The hand soap formulations described herein may be designed to be rinsed off, wiped, off, or left off for maximum cleaning efficiency.

In a preferred embodiment, the tablets are round, however other geometric shapes are contemplated.

### Methods of Using Stable Anhydrous Cleanser Tablets

In one aspect, the invention includes a method of using any of the tablets according to the claimed invention including the steps of (1) filling a bottle or vessel with water, (2) adding a hand soap tablet to the water-filled bottle or vessel, and (3) dissolving the tablet in water by stirring or shaking.

For example, the method of using the effervescent tablet comprises (1) filling a foaming hand soap vessel with water, cold, warm, or hot, (2) add one tablet into the vessel, and (3) placing a foaming pump on the vessel, and (4) dispensing the content in the vessel after the tablet is substantially dissolved.

Each individual hand soap tablet, when exposed to water and stirred or shaken, will dissolve into a liquid cleansing solution. Upon experiencing dissolution of the cleanser tablet, the user may proceed with cleaning or washing as usual. Individual tablets may be packaged together in suitable bulk quantities.

The hand soap tablets may be stored in any suitable container, such as but not limited to plastic, glass, aluminum, ceramic, or acrylic container. The container may contain a desiccant. The container may be re-usable and refilled with new tablets as needed.

### EXEMPLIFICATION

Materials used in the following Examples and their sources are listed below.

### Reference Example 1

A foaming hand soap tablet was produced, using the following ingredients:

**Table 1**

| Raw Materials | Chemistry | Function in tablet | Function in final dilution | Weight (%) |
|---|---|---|---|---|
| Citric Acid | Citric Acid | Acid for effervescent | pH control | 20-30% |
| Sodium Carbonate | Sodium Carbonate Dense | Base for effervescent | Cleaner/pH control | 10-25% |
| Sodium Lauryl Sulfate | Sodium Lauryl Sulfate | Anionic Surfactant | Soil remover | 5-25% |
| BASF Lutensol AT 25 | ethoxylated alcohols | Nonionic Surfactant | oily soil remover | 5-15% |
| PEG 8000 | Polyethylene Glycol 8000 | Binder | solvent | <5% |
| Fragrance | n/a | sensorial effect | sensorial effect | 1-3% |
| Dye/colorant | FD&C or polymetric dye | visual effect | visual effect | 0.001 - 0.01 |
| Total | | | | 100 |
| pH | | | | pH 4.0-6.0 |
| Liquid Load (%) | | | | 3 |

### Example 2

A foaming hand soap tablet was produced, using the following ingredients:

**Table 2**

| **Raw Materials** | **Chemistry** | **Function in tablet** | **Function in final dilution** | **Weight (%)** |
|---|---|---|---|---|
| Citric Acid | Citric Acid | Acid for effervescent | pH control | 25-35 |
| Sodium Coco Sulfate | Sodium Coco Sulfate | Surfactant | oily soil remover | 10-25 |
| Sodium Carbonate | Sodium Carbonate Dense | Base for effervescent | Cleaner/pH control | 10-20 |
| Sodium Benzoate | Sodium Benzoate | Lubricating/Pres ervative | Preservative | 10-30 |
| Sodium Alginate (Alginate SS207) | Sodium Alginate ( | Thickening Agent | emollient | 1-10 |
| PEG 8000 | Polyethylene Glycol 8000 | Binder | solvent | 0-20 |
| Sorbitol Crystalline P20 | Sorbitol | Binder | solvent | 0-10 |
| Potassium Sorbate | Potassium Sorbate | preservative booster | Preservative | 1-10 |
| Fragrance Clean Basil | Fragrance Clean Basil | sensorial effect | sensorial effect | 0-2 |
| Medium-chain triglycerides Oil | Medium-chain triglycerides Oil | Process aid/emollient | emollient | 0.2-0.6 |
| Total | | | | 100.00 |
| pH | | | 4.0-6.0 when dissolved in water | |

One tablet is dissolved into about 266 mL (9 oz) of water.

The cleaning performance of the hand soap formulation described herein are tested using a blind consumer panel. The cleaning efficieny of the hand soap formulation described herein are satisfactory and comparable to that of standard such as dial in terms of cleaning, lathering, rinse off, hand feel, and/or dryness.

## Claims

1. A stable anhydrous hand soap concentrate formulation in a solid form, comprising a surfactant, a preservative, a fragrance, and a pH control agent, wherein the stable anhydrous hand soap concentrate formulation is substantially fatty acid free and/or substantially animal fat free, wherein the preservative is present in an amount ranging from about 10% to about 30%, from about 10% to about 25%, or from about 10% to about 20%, by weight, based on the weight of the formulation; and preferably wherein the preservative is selected from sodium benzoate, gluconolactone, and biocideal preservatives.

2. The stable anhydrous hand soap concentrate formulation of claim 1, wherein the pH control agent is present in an amount that is greater than the amount of the surfactant;
preferably wherein the pH control agent is present in an amount ranging from 1% to about 85%, from about 15% to about 20%, from about 20% to about 60%, from about 20% to about 45%, from about 25% to about 35%, from 5 % to about 40%, from about 5 % to about 30 %, from about 10% to about 30%, from about 10% to about 25%, from about 5% to about 10%, from about 40% to about 60%, from about 35% to about 45%, from about 30 % to about 55 %, or from about 35 % to about 55 %, by weight, based on the weight of the formulation;
and preferably wherein the surfactant is present in an amount ranging from about 0.01% to about 40%, from about 1% to about 20%, from about 10% to about 20%, from about 10% to about 25%, from about 10% to about 40%, from about 5% to about 15 %, from about 5% to about 25%, or from about 15% to about 25%, by weight, based on the weight of the formulation.

3. The stable anhydrous hand soap concentrate formulation of claim 1 or claim 2, wherein the surfactant comprises an anionic and/or nonionic surfactant,
preferably wherein the anionic surfactant is selected from sodium coco sulfate and sodium lauryl sulfate; and
preferably wherein the nonionic surfactant is selected from ethoxylated alcohol and alkyl polyglucosides.

4. The stable anhydrous hand soap concentrate formulation of any of claims 1-3,
wherein the pH control agent comprises an acidic cleaner or basic cleaner, or a combination thereof;
preferably wherein the acidic cleaner is selected from citric acid and malic acid; and
preferably wherein the basic cleaner is selected from sodium carbonate, sodium bicarbonate and any other alkali carbonates.

5. The stable anhydrous hand soap concentrate formulation of any of claims 1-4, further comprising a binding agent.
preferably wherein the binding agent is present in an amount ranging from 0% to about 30%, less than 5%, from about 0% to about 10%, from about 0% to about 20%, from about 3% to about 8%, from about 5% to about 15% by weight, based on the weight of the formulation; and
preferably wherein the binding agent is selected from polyethylene glycol, sorbitol, and dextrose.

6. The stable anhydrous hand soap concentrate formulation of any of claims 1-5, further comprising a thickening agent,
preferably wherein the thickening agent is present in an amount ranging from about 1% to about 15%, from about 1% to about 10%, or from 5% to about 10 %, by weight, based on the weight of the formulation; and
preferably wherein the thickening agent is selected from xanthum gum, NaCl, KCl, potassium alginate, guar gum, and HPMC.

7. The stable anhydrous hand soap concentrate formulation of any of claims 1-6, further comprising a preservative booster,
preferably wherein the preservative booster is present in an amount ranging from about 0.1 % to about 10%, from about 0.5 % to about 10 %, from about 1 % to about 10 %, or from about 1% to about 5%, by weight, based on the weight of the formulation, and
preferably wherein the preservative booster is sorbate.

8. The stable anhydrous hand soap concentrate formulation of any of claims 1-7, further comprising an ingredient selected from process aid, chelating agent, lubricating agent, and a coloring agent.

9. The stable anhydrous hand soap concentrate formulation of any of claims 1-8, wherein said formulation produces a low pH solution in the range of about 4.0 to about 6.0 when dissolved in appropriate amount of water

10. The stable anhydrous hand soap concentrate formulation of any of claims 1-9, comprising citric acid, sodium carbonate, sodium lauryl sulfate, ethoxylated alcohol, polyethylene glycol, and optionally a coloring agent.

11. The stable anhydrous hand soap concentrate formulation of any of claims 1-9, comprising citric acid, sodium carbonate, sodium coco sulfate, sodium benzoate, sodium alginate, polyethylene glycol, sorbitol, medium-chain triglycerides oil, and a fragrance.

12. The stable anhydrous hand soap concentrate formulation of any of claims 1-11, which is in the form of a tablet.

13. The stable anhydrous hand soap concentrate formulation of claim 12, which is in the form of a tablet wherein the tablet is not tacky.

14. A method of preparing a tablet, comprising blending homogeneously the ingredients of any of claims 1-13 to form a mixture and compressing the mixture to form the tablet.

15. A method of using the tablet of claim 13 comprising (1) filling a bottle or vessel with water, (2) adding the tablet to the water-filled bottle or vessel, and (3) dissolving the tablet in appropriate amount of water.

## Patentansprüche

1. Stabile, wasserfreie Handseifenkonzentrat-Formulierung in einer festen Form, umfassend ein grenzflächenaktives Mittel, ein Konservierungsmittel, einen Duftstoff und ein pH-Einstellmittel, wobei die stabile, wasserfreie Handseifenkonzentrat-Formulierung im Wesentlichen fettsäurefrei und/oder im Wesentlichen frei von tierischem Fett ist, wobei das Konservierungsmittel in einer Menge im Bereich von etwa 10 Gew.-% bis etwa 30 Gew.-%, von etwa 10 Gew.-% bis etwa 25 Gew.-% oder von etwa 10 Gew.-% bis etwa 20 Gew.-%, auf der Basis des Gewichts der Formulierung, vorliegt; und
vorzugsweise wobei das Konservierungsmittel aus Natriumbenzoat, Glukonolacton und bioziden Konservierungsmitteln ausgewählt ist.

2. Stabile, wasserfreie Handseifenkonzentrat-Formulierung nach Anspruch 1, wobei das pH-Einstellmittel in einer Menge vorliegt, die größer ist als die Menge des grenzflächenaktiven Mittels;
vorzugsweise wobei das pH-Einstellmittel in einer Menge im Bereich von 1 Gew.-% bis etwa 85 Gew.-%, von etwa 15 Gew.-% bis etwa 20 Gew.-%, von etwa 20 Gew.-% bis etwa 60 Gew.-%, von etwa 20 Gew.-% bis etwa 45 Gew.-%, von etwa 25 Gew.-% bis etwa 35 Gew.-%, von 5 Gew.-% bis etwa 40 Gew.-%, von etwa 5 Gew.-% bis etwa 30 Gew.-%, von etwa 10 Gew.-% bis etwa 30 Gew.-%, von etwa 10 Gew.-% bis etwa 25 Gew.-%, von etwa 5 Gew.-% bis etwa 10 Gew.-%, von etwa 40 Gew.-% bis etwa 60 Gew.-%, von etwa 35 Gew.-% bis etwa 45 Gew.-%, von etwa 30 Gew.-% bis etwa 55 Gew.-% oder von etwa 35 Gew.-% bis etwa 55 Gew.-%, auf der Basis des Gewichts der Formulierung, vorliegt;
und vorzugsweise wobei das grenzflächenaktive Mittel in einer Menge im Bereich von etwa 0,01 Gew.-% bis etwa 40 Gew.-%, von etwa 1 Gew.-% bis etwa 20 Gew.-%, von etwa 10 Gew.-% bis etwa 20 Gew.-%, von etwa 10 Gew.-% bis etwa 25 Gew.-%, von etwa 10 Gew.-% bis etwa 40 Gew.-%, von etwa 5 Gew.-% bis etwa 15 Gew.-%, von etwa 5 Gew.-% bis etwa 25 Gew.-% oder von etwa 15 Gew.-% bis etwa 25 Gew.-%, auf der Basis des Gewichts der Formulierung, vorliegt.

3. Stabile, wasserfreie Handseifenkonzentrat-Formulierung nach Anspruch 1 oder Anspruch 2, wobei das grenzflächenaktive Mittel ein anionisches und/oder nichtionisches grenzflächenaktives Mittel umfasst,
vorzugsweise wobei das anionische grenzflächenaktive Mittel aus Natriumkokossulfat und Natriumlaurylsulfat ausgewählt ist; und
vorzugsweise wobei das nicht-ionische grenzflächenaktive Mittel aus ethoxyliertem Alkohol und Alkylpolyglukosiden ausgewählt ist.

4. Stabile, wasserfreie Handseifenkonzentrat-Formulierung nach einem der Ansprüche 1 bis 3, wobei das pH-Einstellmittel einen sauren Reiniger oder einen basischen Reiniger oder eine Kombination davon umfasst;
vorzugsweise wobei der saure Reiniger aus Zitronensäure und Äpfelsäure ausgewählt ist; und
vorzugsweise wobei der basische Reiniger aus Natriumcarbonat, Natriumhydrogencarbonat und jedweden anderen Alkalicarbonaten ausgewählt ist.

5. Stabile, wasserfreie Handseifenkonzentrat-Formulierung nach einem der Ansprüche 1 bis 4, weiter umfassend ein Bindemittel,
vorzugsweise wobei das Bindemittel in einer Menge im Bereich von 0 Gew.-% bis etwa 30 Gew.-%, weniger als 5 Gew.-%, von etwa 0 Gew.-% bis etwa 10 Gew.-%, von etwa 0 Gew.-% bis etwa 20 Gew.-%, von etwa 3 Gew.-% bis etwa 8 Gew.-%, von etwa 5 Gew.-% bis etwa 15 Gew.-%, auf der Basis des Gewichts der Formulierung, vorliegt; und
vorzugsweise wobei das Bindemittel aus Polyethylenglykol, Sorbit und Dextrose ausgewählt ist.

6. Stabile, wasserfreie Handseifenkonzentrat-Formulierung nach einem der Ansprüche 1 bis 5, weiter umfassend ein Verdickungsmittel,
vorzugsweise wobei das Verdickungsmittel in einer Menge im Bereich von etwa 1 Gew.-% bis etwa 15 Gew.-%, von etwa 1 Gew.-% bis etwa 10 Gew.-% oder von 5 Gew.-% bis etwa 10 Gew.-%, auf der Basis des Gewichts der Formulierung, vorliegt; und
vorzugsweise wobei das Verdickungsmittel aus Xanthangummi, NaCl, KCl, Kaliumalginat, Guargummi und HPMC ausgewählt ist.

7. Stabile, wasserfreie Handseifenkonzentrat-Formulierung nach einem der Ansprüche 1 bis 6, weiter umfassend einen Konservierungsmittel-Wirkungsverstärker,
vorzugsweise wobei der Konservierungsmittel-Wirkungsverstärker in einer Menge im Bereich von etwa 0,1 Gew.-% bis etwa 10 Gew.-%, von etwa 0,5 Gew.-% bis etwa 10 Gew.-%, von etwa 1 Gew.-% bis etwa 10 Gew.-% oder von etwa 1 Gew.-% bis etwa 5 Gew.-%, auf der Basis des Gewichts der Formulierung, vorliegt, und
vorzugsweise wobei der Konservierungsmittel-Wirkungsverstärker Sorbat ist.

8. Stabile, wasserfreie Handseifenkonzentrat-Formulierung nach einem der Ansprüche 1 bis 7, weiter umfassend einen Bestandteil, der aus Verarbeitungshilfsmittel, Chelatisierungsmittel, Schmiermittel und einem Farbmittel ausgewählt ist.

9. Stabile, wasserfreie Handseifenkonzentrat-Formulierung nach einem der Ansprüche 1 bis 8, wobei die Formulierung eine Lösung mit niedrigem pH-Wert im Bereich von etwa 4,0 bis etwa 6,0 erzeugt, wenn sie in einer geeigneten Menge Wasser gelöst wird.

10. Stabile, wasserfreie Handseifenkonzentrat-Formulierung nach einem der Ansprüche 1 bis 9, umfassend Zitronensäure, Natriumcarbonat, Natriumlaurylsulfat, ethoxylierten Alkohol, Polyethylenglykol und gegebenenfalls ein Farbmittel.

11. Stabile, wasserfreie Handseifenkonzentrat-Formulierung nach einem der Ansprüche 1 bis 9, umfassend Zitronensäure, Natriumcarbonat, Natriumkokossulfat, Natriumbenzoat, Natriumalginat, Polyethylenglykol, Sorbit, Triglyceridöl mit mittlerer Kettenlänge und einen Duftstoff.

12. Stabile, wasserfreie Handseifenkonzentrat-Formulierung nach einem der Ansprüche 1 bis 11, die in der Form einer Tablette vorliegt.

13. Stabile, wasserfreie Handseifenkonzentrat-Formulierung nach Anspruch 12, die in der Form einer Tablette vorliegt, wobei die Tablette nicht klebrig ist.

14. Verfahren zur Herstellung einer Tablette, umfassend das homogene Mischen der Bestandteile nach einem der Ansprüche 1 bis 13 zur Bildung eines Gemischs und das Pressen des Gemischs zur Bildung der Tablette.

15. Verfahren zur Verwendung der Tablette nach Anspruch 13, umfassend (1) das Füllen einer Flasche oder eines Behälters mit Wasser, (2) das Hinzufügen der Tablette zu der wassergefüllten Flasche oder dem wassergefüllten Behälter und (3) das Lösen der Tablette in einer geeigneten Menge Wasser.

## Revendications

1. Formulation stable et anhydre de concentré de savon pour les mains sous une forme solide, comprenant un tensioactif, un conservateur, un parfum et un agent de régulation du pH, dans laquelle la formulation stable et anhydre de concentré de savon pour les mains est sensiblement exempte d'acides gras et/ou sensiblement exempte de graisses animales, dans laquelle le conservateur est présent en une quantité allant d'environ 10 % à environ 30 %, d'environ 10 % à environ 25 %, ou d'environ 10 % à environ 20 %, en poids, sur la base du poids de la formulation ; et
de préférence dans laquelle le conservateur est choisi parmi le benzoate de sodium, la gluconolactone et les conservateurs biocides.

2. Formulation stable et anhydre de concentré de savon pour les mains selon la revendication 1, dans laquelle l'agent de régulation du pH est présent en une quantité supérieure à la quantité du tensioactif ;
de préférence dans laquelle l'agent de régulation du pH est présent en une quantité allant de 1 % à environ 85 %, d'environ 15 % à environ 20 %, d'environ 20 % à environ 60 %, d'environ 20 % à environ 45 %, d'environ 25 % à environ 35 %, de 5 % à environ 40 %, d'environ 5 % à environ 30 %, d'environ 10 % à environ 30 %, d'environ 10 % à environ 25 %, d'environ 5 % à environ 10 %, d'environ 40 % à environ 60 %, d'environ 35 % à environ 45 %, d'environ 30 % à environ 55 %, ou d'environ 35 % à environ 55 %, en poids, sur la base du poids de la formulation ;
et de préférence dans laquelle le tensioactif est présent en une quantité allant d'environ 0,01 % à environ 40 %, d'environ 1 % à environ 20 %, d'environ 10 % à environ 20 %, d'environ 10 % à environ 25 %, d'environ 10 % à environ 40 %, d'environ 5 % à environ 15 %, d'environ 5 % à environ 25 %, ou d'environ 15 % à environ 25 %, en poids, sur la base du poids de la formulation.

3. Formulation stable et anhydre de concentré de savon pour les mains selon la revendication 1 ou la revendication 2, dans laquelle le tensioactif comprend un tensioactif anionique et/ou non ionique,
de préférence dans laquelle le tensioactif anionique est choisi parmi le coco-sulfate de sodium et le laurylsulfate de sodium ; et
de préférence dans laquelle le tensioactif non ionique est choisi parmi l'alcool éthoxylé et les polyglucosides d'alkyle.

4. Formulation stable et anhydre de concentré de savon pour les mains selon l'une quelconque des revendications 1 à 3, dans laquelle l'agent de régulation du pH comprend un agent nettoyant acide ou un agent nettoyant basique, ou une combinaison de ceux-ci ;
de préférence dans laquelle l'agent nettoyant acide est choisi parmi l'acide citrique et l'acide malique ; et
de préférence dans laquelle l'agent nettoyant basique est choisi parmi le carbonate de sodium, le bicarbonate de sodium et tous autres carbonates alcalins.

5. Formulation stable et anhydre de concentré de savon pour les mains selon l'une quelconque des revendications 1 à 4, comprenant en outre un agent liant.
de préférence dans laquelle l'agent liant est présent en une quantité allant de 0 % à environ 30 %, inférieure à 5 %, d'environ 0 % à environ 10 %, d'environ 0 % à environ 20 %, d'environ 3 % à environ 8 %, d'environ 5 % à environ 15 % en poids, sur la base du poids de la formulation ; et
de préférence dans laquelle l'agent liant est choisi parmi le polyéthylèneglycol, le sorbitol et le dextrose.

6. Formulation stable et anhydre de concentré de savon pour les mains selon l'une quelconque des revendications 1 à 5, comprenant en outre un agent épaississant,
de préférence dans laquelle l'agent épaississant est présent en une quantité allant d'environ 1 % à environ 15 %, d'environ 1 % à environ 10 %, ou de 5 % à environ 10 %, en poids, sur la base du poids de la formulation ; et
de préférence dans laquelle l'agent épaississant est choisi parmi la gomme xanthane, le NaCl, le KCl, l'alginate de potassium, la gomme de guar et l'HPMC.

7. Formulation stable et anhydre de concentré de savon pour les mains selon l'une quelconque des revendications 1 à 6, comprenant en outre un potentialisateur de conservateur,
de préférence dans laquelle le potentialisateur de conservateur est présent en une quantité allant d'environ 0,1 % à environ 10 %, d'environ 0,5 % à environ 10 %, d'environ 1 % à environ 10 %, ou d'environ 1 % à environ 5 %, en poids, sur la base du poids de la formulation, et
de préférence dans laquelle le potentialisateur de conservateur est du sorbate.

8. Formulation stable et anhydre de concentré de savon pour les mains selon l'une quelconque des revendications 1 à 7, comprenant en outre un ingrédient choisi parmi un auxiliaire de fabrication, un agent chélatant, un agent lubrifiant et un agent colorant.

9. Formulation stable et anhydre de concentré de savon pour les mains selon l'une quelconque des revendications 1 à 8, dans laquelle ladite formulation produit une solution à faible pH dans la plage allant d'environ 4,0 à environ 6,0 lorsqu'elle est dissoute dans une quantité appropriée d'eau

10. Formulation stable et anhydre de concentré de savon pour les mains selon l'une quelconque des revendications 1 à 9, comprenant de l'acide citrique, du carbonate de sodium, du laurylsulfate de sodium, de l'alcool éthoxylé, du polyéthylèneglycol et éventuellement un agent colorant.

11. Formulation stable et anhydre de concentré de savon pour les mains selon l'une quelconque des revendications 1 à 9, comprenant de l'acide citrique, du carbonate de sodium, du coco-sulfate de sodium, du benzoate de sodium, de l'alginate de sodium, du polyéthylèneglycol, du sorbitol, de l'huile de triglycérides à chaîne moyenne et un parfum.

12. Formulation stable et anhydre de concentré de savon pour les mains selon l'une quelconque des revendications 1 à 11, qui se présente sous la forme d'un comprimé.

13. Formulation stable et anhydre de concentré de savon pour les mains selon la revendication 12, qui se présente sous la forme d'un comprimé, dans laquelle le comprimé n'est pas collant.

14. Procédé de préparation d'un comprimé, comprenant le mélange homogène des ingrédients de l'une quelconque des revendications 1 à 13 pour former un mélange et la compression du mélange pour former le comprimé.

15. Procédé d'utilisation du comprimé selon la revendication 13, comprenant (1) le remplissage d'une bouteille ou d'un récipient avec de l'eau, (2) l'ajout du comprimé à la bouteille ou au récipient rempli d'eau, et (3) la dissolution du comprimé dans une quantité appropriée d'eau.
